# EUROPEAN PATENT APPLICATION

(11) **EP 1 980 624 A2**
(43) Date of publication of application: **15.10.2008**
(21) Application number: 06841756.7
(22) Date of filing: 19.12.2006
(51) Int. Cl.: C12Q 1/68

(54) **METHOD FOR THE ANALYSIS OF DIFFERENTIAL EXPRESSION IN COLORECTAL CANCER**

(30) Priority: 21.12.2005 ES 200503203
(71) Applicant: Oryzon Genomics, S.A., 08028 Barcelona (ES)
(72) Inventor: BUESA ARJOL, Carlos, E-08028 Barcelona (ES); SCHWARTZ NAVARRO, Simó, E-08028 Barcelona (ES); ARANGO DEL CORRO, Diego, E-08028 Barcelona (ES)
(74) Representative: Barlocci, Anna
(86) International application number: PCT/ES2006/000704
(87) International publication number: WO 2007/074193

(57) **Abstract**

A method for the analysis of differential expression in colorectal cancer based on the variation in the expression levels of genes encoding for proteins forming part of the condensin complex or associated proteins that occurs in patients with the said disease and that can be used as markers for the diagnosis of the said cancers, as well as for the prevention and treatment thereof.

## Description

### FIELD OF THE INVENTION

The present invention relates to a method for the analysis of differential expression, based on the overexpression of proteins of the condensin complex and associated proteins in colorectal cancer patients, which can be used as a criterion for the diagnosis of said cancers as well as in the prevention and treatment thereof.

### BACKGROUND OF THE INVENTION

In absolute terms, cancer is the second most common cause of death in Spain. Among the many types of cancer, colorectal cancer stands out, according to 1999 data, as having been responsible for 11% of cancer deaths in men and 15% in women. In Spain, the estimated annual number of new cases of both sexes stands at around 18,000, as against 11,300 deaths. Owing to frequent errors in classifying tumors of the rectosigmoid portion, colon and rectal tumors are generally treated as one for analysis purposes. Mortality from colorectal cancer is very high, this being the second most common form of cancer in both men and women, with the trend rising with age (2.2% annually in men and 0.7% in women). Nowadays mortality is higher in men, though in the 1960s it was higher in women.

With these tumors, mortality data do not reflect the true incidence of the disease, given that survival has improved in recent years, primarily in young people. The trend toward stabilization of mortality may reflect the therapeutic improvements achieved with early diagnosis, given that the tumors in question are fairly accessible to sigmoidoscopic examination and the universal use of full colonoscopies for screening identified risk groups.

The cumulative lifetime risk of contracting the disease is 5-6%, depending on lifestyles and hereditary factors. The most common colorectal cancer is the sporadic type (90%), and some cases having elements of inherited predisposition: familial adenomatous polyposis (0.01%) and hereditary nonpolyposis colorectal cancer (5-10%). The latter are caused by familial syndromes defined by just a few genes. However, the genes responsible for sporadic cancers have yet to be identified. It is believed that colorectal tumor may develop as a consequence of a series of molecular events that start off with one or more mutations or epigenetic events and continue with progression phenomena in which both genetic and environmental factors may be involved.

Generally speaking, with colorectal cancer the symptoms usually only become apparent in the advanced stages of growth in the intestinal wall, which makes it necessary to identify new genes and/or mutations responsible for, and/or indicative of this type of cancer and to stimulate the consequent development of analytical methods that will allow selective and rapid molecular diagnosis of the disease in order that they can eventually form part of routine clinical practice.

This would also lead to savings in healthcare costs and reduced waiting times, to the adoption of new prognostic criteria and treatment approaches in positive cases, and to the design of selective therapies associated with these genes.

The protein encoded by the gene SMC2L1 (human ortholog of SMC2 of S. *cerevisiae*, also called hCAP-E) is a crucial protein in chromosome condensation and compaction complexes that could perform functions related to the regulation of gene transcription (Hagstrom and Meyer, 2003; Hirano, 2002; Legagneux et al., 2004).

This protein belongs to a family of so-called SMC proteins (standing for "structural maintenance of chromosomes"), which comprises 6 proteins numbered from 1 to 6 (SMC1, SMC2, SMC3, SMC4, SMC5, and SMC6). These proteins form dimers with other SMC proteins and active complexes with other so-called non-SMC proteins (divided into two families, HEAT and kleisin). Overall, they form three primary complexes: condensin (in humans there are two differentiated complexes, condensin I and condensin II), cohesin and the hSMC5-hSMC6 complex, associated with genomic repair (Hagstrom and Meyer, 2003; Hirano, 2002). The proteins and the complexes they form in different species are described in Tables 1 and 2.

**Table 1. Structural maintenance of chromosomes**

| | **Saccharomyces cerevisiae** | **Schizosaccharomyces pombe** | **Caenorhabditis elegans** | **Drosophila melanogaster** | **Xenopus laevis** | **Homo sapiens** |
|---|---|---|---|---|---|---|
| **Cohesin** | | | | | | |
| SMC1 | Smc1 | Psm1 | HIM-1 | SMC1 | SMC1 | SMC1 |
| SMC3 | Smc3 | Psm3 | SMC-3 | SMC3 | SMC3 | SMC3 |
| SCC1 | Scc1/Mcd1 | Rad21 | SCC-1/COH-2 | Rad21 | RAD21 | RAD21 |
| SCC3 | Scc3 | Psc3 | SCC-3 | SA | SA1,SA2 | SA1,SA2 |
| | | | | | | |

| **Condensin** | | | | | | |
|---|---|---|---|---|---|---|
| SMC2 | Smc2 | Cut14 | MIX-1 | SMC2 | CAP-E | CAP-E |
| SMC4 | Smc4 | Cut3 | SMC-4 | SMC4/gluon | CAP-C | CAP-C |
| CAP-D2 | Ycs4 | Cnd1 | HCP-6 | CG1911 | CAP-D2 | CAP-D2 |
| CAP-G | Ycs5/Ycg1 | Cnd3 | | CG17054 | CAP-G | CAP-G |
| CAP-H | Brn1 | Cnd2 | DPY-26 | Barren | CAP-H | CAP-H |
| | | | | | | |

| **DNA repair** | | | | | | |
|---|---|---|---|---|---|---|
| SMC5 | Smc5 | Spr18 | C27A2.1 | CG3248 | SMC5 | SMC5 |
| SMC6 | Smc6/Rhc18 | Rad18 | C23H4.6 | CG5524 | SMC6 | SMC6 |

(Adapted from Hagstrom and Meyer, 2003)

**Table 2. Condensin complexes**

| | SMC subunits | | HEAT-repeat subunits | | Kleisin subunits |
|---|---|---|---|---|---|
| Condensin *S. cerevisiae* | Smc2 | Smc4 | Ycs4p | Ycs5p/Ycg1p | Brn1 |
| Condensin *S. pombe* | Cut14 | Cut3 | Cnd1 | Cnd3 | Cnd2 |
| Condensin I *D. melanogaster* | SMC2 | SMC4/Gluon | CG1911 | CG 17054 | Barren |
| | | | | | |
| Condensin I *C. elegans* | MIX-1 | DPY-27 | DPY-28 | ? | DPY-26 |
| Condensin I C. elegans | MIX-1 | SMC-4 | HCP-6 | ? | KLE-2/C29E4.2 |
| Condensin I *X. laevis* | XCAP-E | XCAP-C | XCAP-D2 | XCAP-G | XCAP-H |
| Condensin II *X. laevis* | XCAP-E | XCAP-C | XCAP-D3 | XCAP-G2 | XCAP-H2 |
| | | | | | |
| Condensin I *H. sapiens* | hCAP-E | hCAP-C | hCAP-D2 | hCAP-G | hCAP-H |
| Condensin II *H. sapiens* | hCAP-E | hCAP-C | hCAP-D3 | hCAP-G2 | hCAP-H2 |

SMC2L1 forms part of the condensin I and II complexes. Both complexes perform functions relating to chromatin compaction (Hirano et al., 1994; Ono et al., 2004; Ono et al., 2003):
Condensin I: formed by the dimer SMC2-SMC4 (hCAP-E-hCAP-C), the HEAT subunits hCAP-D2/CNAP1 and hCAP-G, and the kleisin subunit hCAP-H. The dimer hCAP-E-hCAP-C is located in the cytoplasm of interphase cells (except during mitosis). Once mitosis has occurred, the non-SMC subunits undergo phosphorylation, interact with the hCAP-E-hCAP-C dimer, form the complex, and are transferred to the nucleus, where they interact with the DNA and compact it (Hagstrom and Meyer, 2003).
Condensin II: formed by the dimer SMC2-SMC4 (hCAP-E-hCAP-C), HEAT subunits hCAP-D3 and hCAP-G, and kleisin subunit hCAP-H2. It seems that the complex is located in the nucleus of interphase cells (Fig. 1A) and could be associated with the regulation of gene transcription, helping to compact the promoter region of certain genes. This would place condensin in the euchromatin regions associated with transcriptionally active regions (regions of transcriptional regulation, i.e. of transcription activation or inhibition), as the regions equivalent to the internal nuclear region (Fig. 1A and 1B) and the chromosome R-bands, with other regulatory factors such as acetylated histones (for example, acetylated histone H3 at lysine K3) (Figs 2 and 3).

There is no evidence in the literature relating these proteins with this type of cancer. However, the possible link between condensin and transcriptional silencing and proper chromosome compaction suggests that it could be altered in cancer. Most cancers present general genomic hypomethylation accompanied by general hyperacetylation, but it has been shown that certain gene promoter regions rich in CpG islands are hypermethylated and the genes are silenced (are not transcribed). In the present invention it is described how condensin is involved in these silencing processes and how the proteins forming these complexes and associated proteins are overexpressed. Similarly, given that it is believed that the majority of epigenetic alterations occur in the very early stages of neoplastic processes, this overexpression may also be characteristic of early processes such as adenomas.

### DESCRIPTION OF THE INVENTION

It is the object of the present invention to provide a method for the analysis of differential expression in colorectal cancer, comprising the determination, in a biological sample isolated from a patient, of a variation in the expression levels of one or more protein-encoding genes forming part of the condensin complex or other proteins interacting with the said complex, where the said variation in gene expression levels is used to diagnose for the presence of colorectal cancer or of a premalignant condition thereof.

In particular, the gene or genes to be analyzed are selected from among the group made up of hCap-E, hCap-C, hCap-D2, hCap-D3, hCap-G, hCap-G2, hCap-H, hCap-H2, and KIF4A and the variation in the expression levels of the gene or genes is an increase in expression levels.

In particular, the said sample may be DNA or RNA and may be isolated from cells obtained by biopsy or any other method of extraction.

In an embodiment of the invention, the determination is carried out by PCR amplification, SDA amplification, or any other method of nucleic acid amplification.

In another embodiment of the invention, the determination may be carried out by means of DNA biochips made with oligonucleotides deposited by any mechanism or by means of DNA biochips made with oligonucleotides synthesized in situ by photolithography or any other mechanism.

In another embodiment of the invention, the determination is carried out by in situ hybridization using specific probes labeled using any labeling method.

In another embodiment of the invention, the determination is carried out by gel electrophoresis. Optionally, the determination may be carried out by transfer to a membrane and hybridization with a specific probe.

In another embodiment of the invention, the determination is carried out by NMR or any other diagnostic imaging technique.

In another embodiment of the invention, the determination is carried out by NMR or any other diagnostic imaging technique and the use of paramagnetic nanoparticles or any other type of detectable nanoparticles functionalized with antibodies or any other means.

Optionally, the sample analyzed may be the protein encoded by the gene or fragments thereof.

In another embodiment of the invention, the determination is carried out by incubation with a specific antibody. Optionally, determination may be carried out by Western blot or by immunohistochemistry.

In another embodiment of the invention, the determination is carried out by protein gel electrophoresis.

In another embodiment of the invention, the determination is carried out using protein chips.

In another embodiment of the invention, the determination is carried out by ELISA or any other enzymatic method.

It is also an object of the present invention to provide a method for the analysis of differential expression in colorectal cancer, wherein the variation in the expression levels of one or more of the described genes is used for predicting the progression of the colorectal cancer or of a premalignant condition thereof, or for predicting the risk of recurrence of the said disease.

It is also an object of the present invention to provide a kit for carrying out the method for the analysis of differential expression, comprising the requisite reagents and additives for determining the variation in the levels of expression of the gene or genes.

It is also an additional object of the present invention to provide a method for the analysis of compounds with therapeutic potential in colorectal cancer, comprising the determination of the capacity of said compounds to decrease the expression levels of one or more of the described genes, with the compounds being compounds tailored according to the sequence information, such as antisense or RNA interference oligonucleotides or others based on the destabilization and elimination of the mRNA or the lack of its translation into protein.

It is also an additional object of the present invention to provide a method for the analysis of compounds with therapeutic potential in colorectal cancer, comprising the determination of the capacity of said compounds to counteract the variation in the levels of expression of one or more of the described genes, with the compounds being paramagnetic nanoparticles or thermally-excited nanoparticles.

It is also an additional object of the present invention to provide a method for the analysis of compounds with therapeutic potential in colorectal cancer, comprising the determination of the capacity of said compounds to counteract the variation in the levels of expression of one or more of the described genes, with the compounds being nanoparticles functionalized with specific antibodies and toxic compounds conveyed in a simple, binary, or modular manner toward the malignant cell.

It is likewise an object of the present invention to provide a pharmaceutical preparation comprising an effective amount of compounds with therapeutic potential identified according to the methods described above and one or more pharmaceutically acceptable excipients.

In addition, it is also an object of the present invention to use compounds with therapeutic potential obtained according to the methods described above for the preparation of a medicinal product for the treatment or prevention of colorectal cancer or of a premalignant condition thereof.

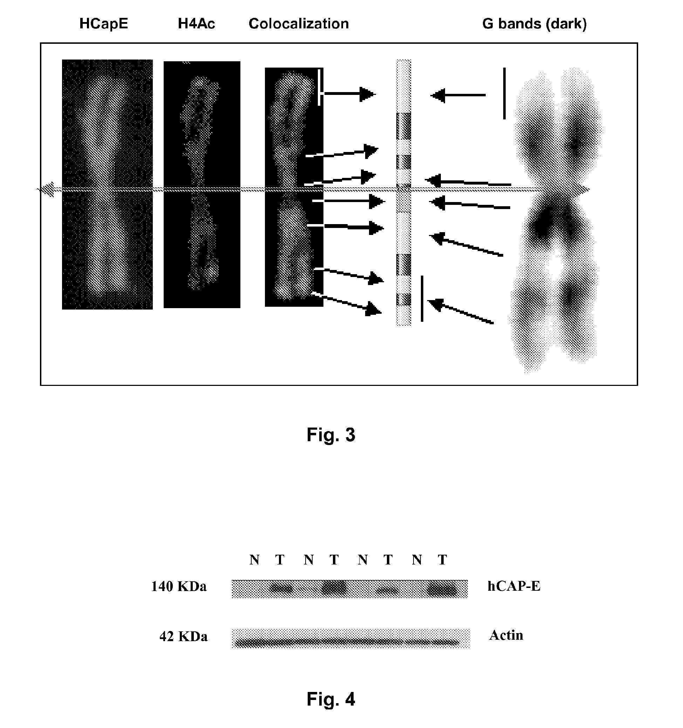

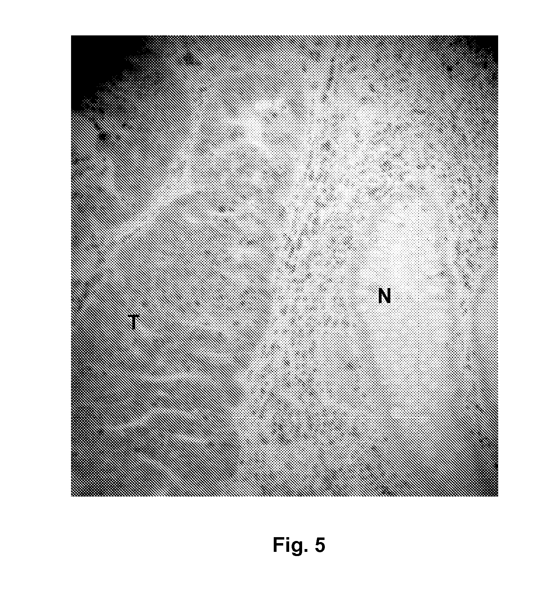

The present invention is based on the overexpression of proteins of the condensin complex and associated proteins observed in colorectal cancer patients. The data showed an overexpression of hCAP-E in the Western blot analysis using specific anti-hCAP-E antibodies in colorectal cancer samples in comparison with samples of normal tissue, regardless of its tumor stage, with a 90% incidence of tumor overexpression (18/20) (Fig. 4). This overexpression was also observed in all immunohistochemical analyses of colorectal cancer tumor tissue (Fig. 5).

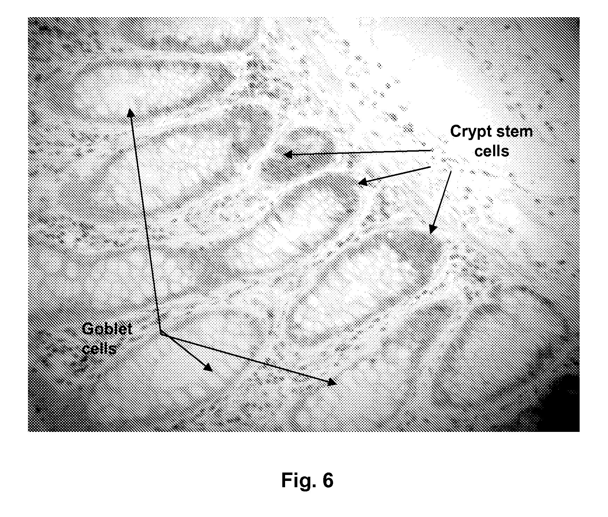

Likewise it was observed that the expression of hCAP-E is also specific for pluripotent (stem) cells of the colon crypt (Fig. 6), which are the undifferentiated cells from which colorectal tumors originate. Their expression pattern indicates that the latter virtually disappears as the pluripotent cells are transformed into epithelial cells (goblet cells) in a normal crypt to form the epithelium, so that it could be regarded as a marker of cell differentiation.

Table 3 shows the levels of overexpression of hCAP-E in colorectal cancer according to the staging.

**Table 3. Overexpression of hCAP-E in colorectal cancer according to the staging**

| **CASE** | **STAGE** | **hCAP-E** |
|---|---|---|
| 94T | I | + |
| 78T | II | + |
| 100T | II | + |
| 67T | II | + |
| 85T | II | + |
| 60T | III | + |
| 162T | III | + |
| 141T | III | + |
| 38T | III | + |
| 55T | III | + |
| 66T | III | - |
| 88T | III | + |
| 91T | III | + |
| 213T | IV | = |
| 129T | IV | + |
| 35T | IV | + |
| 31T | IV | + |
| 36T | IV | + |
| 86T | IV | + |
| 137T | IV | + |

The incidence of overexpression (+) was 90% (18 out of 20 tumors). Only one case was observed in which expression was below normal (-) and one case in which normal tissue and tumor tissue showed similar expression levels (=).

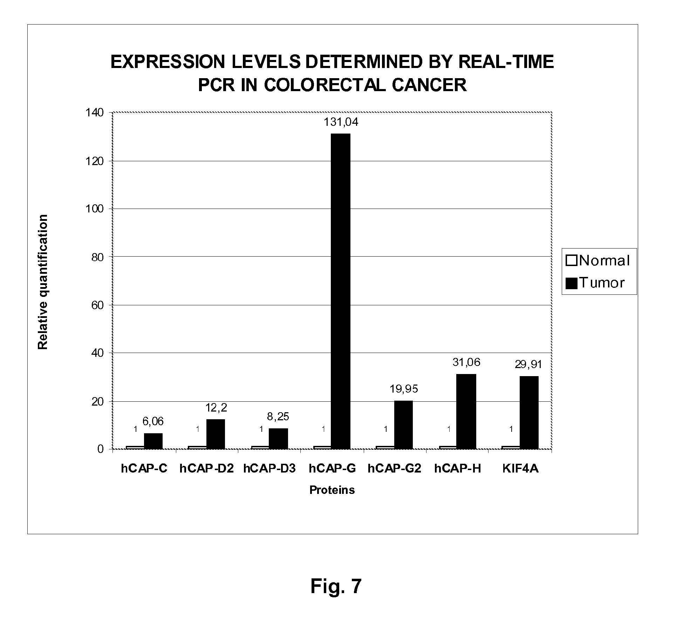

The expression levels of other proteins forming the condensin complex, such as hCap-C, hCap-D2, hCap-D3, hCap-G, hCap-G2 and hCap-H, were likewise analyzed by real-time PCR, as were other associated proteins that interact with the complex, such as KIF4A, in certain tumors in which overexpression of hCap-E had been observed. The results indicated that all the analyzed proteins showed increased expression levels in tumor tissue in comparison with the levels in normal tissue (Fig. 7). It may therefore be concluded that all the proteins that make up the condensin complex and other proteins interacting with the said complex are overexpressed in tumors.

Accordingly, the proteins forming the condensin complex and other associated proteins that interact with it may be used as markers of colorectal cancer or of a premalignant condition thereof, potentially acting as a diagnostic marker and/or a marker for recommending colonoscopy.

These proteins can also act both as markers of pluripotent stem cells of the colon crypt and as markers of cell differentiation. Moreover, these proteins can be histological markers of cancer and/or be useful in imaging analysis systems.

In addition, these proteins can constitute direct or indirect therapeutic targets, enabling tumor-targeted anticancer treatments to hit the tumor through interactions with any of these proteins or by modulation of their expression levels.

Interestingly, it has recently been reported that the expression levels of the proteins in the condensin complex do not vary throughout the cell cycle and, therefore, do not vary during mitosis (Takemoto et al., 2004). So, the fact that high levels of these proteins are found in cancer cells, as described in the present invention, cannot be attributed simply to an increase in replication activity (mitosis) of the tumor cell, but rather to actual relative overexpression due to the development of the disease.

Therefore, the present invention shows that there is a complete association between the expression levels of the proteins that make up the condensin complex and other proteins associated with the said complex and the presence of colorectal cancer, whatever its stage of development, which means that there is now a new molecular tool available that enables the disease to be diagnosed even in its earliest stages, something that is not possible using methods currently available.

### REFERENCES

- Hagstrom K, Meyer BJ. Condensin and cohesin: more than chromosome compactor and glue. Nat Rev Genet 2003 July 4:520-534.
- Hirano T. The ABC of SMC proteins: two-armed ATPases for chromosome condensation, cohesion and repair. Genes & Development, 2002 16:399-341.
- Legagneux V, Cubizolles F, Watrin E. Multiple roles of condensins: a complex story. Biology of the Cell, 2004 96:201-213.
- Hirano T, Mitchison TJ. A heterodimeric coiled-coil protein required for mitotic chromosome condensation in vitro. Cell 1994 Nov 4;79(3):449-58.
- Ono T, Fang Y, Spector DL, Hirano T. Spatial and temporal regulation of Condensins I and II in mitotic chromosome assembly in human cells. Mol Biol Cell. 2004 Jul;15(7):3296-308.
- Ono T, Losada A, Hirano M, Myers MP, Neuwald AF, Hirano T. Differential contributions of condensin I and condensin II to mitotic chromosome architecture in vertebrate cells. Cell. 2003 Oct 3;115(1):109-21.
- Takemoto A, Kimura K, Yokoyama S, Hanaoka F. Cell cycle-dependent phosphorylation, nuclear localization, and activation of human condensin. J Biol Chem. 2004 Feb 6;279(6):4551-9.

### BRIEF DESCRIPTION OF THE FIGURES

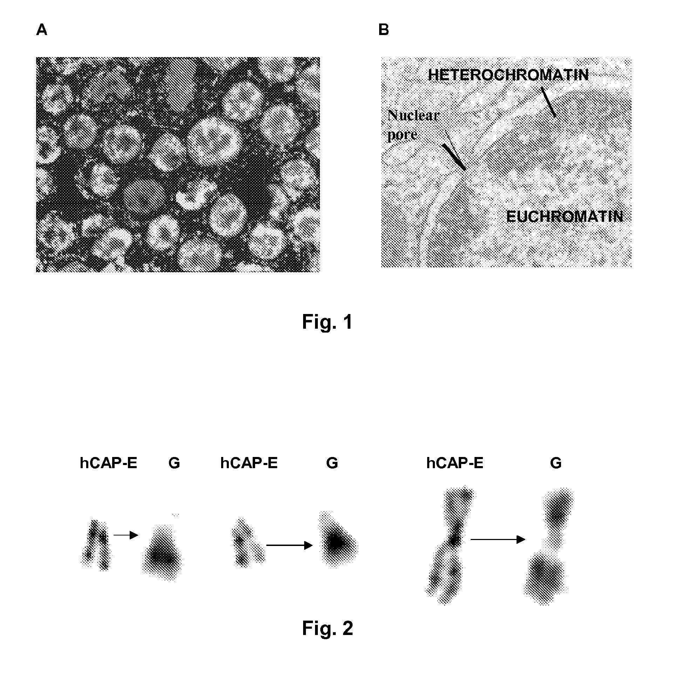

Figure 1A shows hCAP-E localized in interphase cells, represented by the whitish clusters. Figure 1B is an electron microscopic photograph of a cell nucleus separately showing the heterochromatin and the euchromatin.
Figure 2 shows the colocalization of hCAP-E in chromosomal regions equivalent to euchromatin (R bands). The same chromosome is shown stained with hCAP-E and stained for G bands. Heterochromatin is identifiable by the darker areas, the one on the right in each of the figures.
Figure 3 shows the colocalization of hCAP-E with acetylated histone 4 (H4Ac) and the R bands in metaphase chromosomes (light bands in the chromosome).
Figure 4 shows the results of the Western blot analysis of hCAP-E in normal samples (N) and samples taken from the colorectal cancer tumor (T). Actin was used as loading control.
Figure 5 relates to an immunohistochemical analysis of colorectal tissue using a specific anti-hCAP-E antibody. The picture shows the area corresponding to the normal crypt (N) and the area corresponding to the colon adenocarcinoma (T), which exhibits greater expression of hCAP-E.
Figure 6 relates to an immunohistochemical analysis in normal colon crypts using a specific anti-hCAP-E antibody, in which it is observed that the pluripotent stem cells exhibit a stronger specific staining for hCAP-E than for goblet cells.
Figure 7 shows the results of the real-time PCR analysis of other proteins in the condensin complex and the associated protein KIF4A. The sample analyzed was sample 67T, which had previously been observed, when compared to its corresponding normal sample, to overexpress hCAP-E. The analysis was carried out in triplicate and ribosomal 18S was used as internal control.

### DESCRIPTION OF A PREFERRED EMBODIMENT

Below is described a preferred, though not exclusive, embodiment of the invention.

### Patient samples

Biopsies of normal and cancerous tissue were obtained from 20 patients diagnosed with colorectal cancer. The surgically obtained samples were immediately frozen in liquid nitrogen and kept at -80°C for later extraction of proteins and RNA. In addition, histological sections were prepared for immunohistochemical testing. The clinicopathological characteristics were recorded, including the stage and differentiation grade of the tumors, as well as at least a three-year follow-up to detect any early recurrence.

### Analysis of the expression levels of hCAP-E

**Western blot:** The proteins were extracted from the samples of normal and colorectal tissue by standard methods, using RIPA lysis buffer. 90 µg of protein were fractionated in 10% SDS-PAGE gel, transferred to a nitrocellulose membrane (BioRAD, USA), blocked with 5% milk in TBS-T, and hybridized with a primary anti-hCAP-E antibody (Abcam, UK) diluted 1:2500 in blocking solution, and then with a secondary anti-rabbit antibody (Dako Cytomation, Denmark) in a 1:500 dilution.The chemiluminescent signal was detected using the ECL kit (Amersham, USA) and the expression levels of the tumor samples were compared with their normal counterparts. Finally, the membrane was rehybridized with anti-actin antibody (Invitrogen, USA), which was used as load control. As can be seen from Figure 4, all the tumor samples exhibited increased hCAP-E expression levels.

**Immunohistochemistry:** The histological sections taken from the tissues of patients with colorectal cancer were deparaffinized with xylene and rinsed in decreasing series of ethanol and distilled water. The sections were treated with citrate buffer at pH 6 (5 min at 800W and 10 min at 450W in a microwave) the endogenous peroxidase was blocked with H₂O₂, and they were then hybridized with the primary anti-hCAP-E antibody (Abcam, UK). For the immunohistochemical analysis, the EnVision + Dual Link System kit (Dako Cytomation, Denmark) was used in accordance with the manufacturer's recommendations. Finally, the hCAP-E expression levels in areas of normal and cancerous tissue were compared. Figure 5 shows that hCAP-E expression is greater in cancerous than in normal tissue.

### Analysis of the expression levels of other proteins of the condensin complex and associated proteins

**Real-time PCR:** The mRNA levels corresponding to other proteins of the condensin complex and associated proteins were quantified by real-time PCR, for which purpose RNA was extracted from samples of normal and colorectal tissue kept at -80°C, using Trizol (Invitrogen, USA). 10 µg of RNA was retrotranscribed using the High Capacity cDNA Archive kit (Applied Biosystems, USA) and amplified with TaqMan® Gene Expression Assays (Applied Biosystems, USA) for hCAP-C, hCAP-D2, hCAP-D3, hCAP-G, hCAP-G2, hCAP-H, and KIF4A, respectively. The amplification reaction was carried out using TaqMan Universal PCR Master Mix in the 7500 Real-Time PCR System (both from Applied Biosystems, USA). The relative mRNA levels of each gene were quantified using the ΔΔC_{T} method and the program associated with the system. The test was carried out in triplicate and 18S rRNA was used as the endogenous control, and the expression of normal tissue and of cancerous tissue from the same patient was compared. As Figure 7 shows, for all the genes analyzed, the cancerous samples exhibited substantially elevated mRNA levels when compared to the control samples.

## Claims

1. A method for the analysis of differential expression in colorectal cancer, comprising the determination in a biological sample isolated from a patient of variations in the expression levels of one or more protein-encoding genes forming part of the condensin complex or other proteins interacting with the said complex, where the said variation in the gene expression levels is used to diagnose for the presence of colorectal cancer or of a premalignant condition thereof.

2. The method for the analysis of differential expression in colorectal cancer as claimed in claim 1, wherein the gene or genes are selected from among the group comprising hCap-E, hCap-C, hCap-D2, hCap-D3, hCap-G, hCap-G2, hCap-H, hCap-H2, and KIF4A.

3. The method for the analysis of differential expression in colorectal cancer as claimed in any one of claims 1 to 2, wherein the variation in the expression levels of the gene or genes is an increase in expression levels.

4. The method for the analysis of differential expression in colorectal cancer as claimed in any one of claims 1 to 3, wherein the sample is isolated from cells obtained by biopsy or any other method of extraction.

5. The method for the analysis of differential expression in colorectal cancer as claimed in any one of claims 1 to 4, wherein the sample to be analyzed is DNA.

6. The method for the analysis of differential expression in colorectal cancer as claimed in any one of claims 1 to 4, wherein the sample to be analyzed is RNA.

7. The method for the analysis of differential expression in colorectal cancer as claimed in any one of claims 1 to 6, wherein the determination is carried out by PCR amplification, SDA amplification, or any other method of nucleic acid amplification.

8. The method for the analysis of differential expression in colorectal cancer as claimed in any one of claims 1 to 6, wherein the determination is carried out using DNA biochips made with oligonucleotides deposited by any mechanism.

9. The method for the analysis of differential expression in colorectal cancer as claimed in any one of claims 1 to 6, wherein the determination is carried out using DNA chips made with oligonucleotides synthesized in situ by photolithography or by any other mechanism.

10. The method for the analysis of differential expression in colorectal cancer as claimed in any one of claims 1 to 6, wherein the detection is performed by in situ hybridization using specific probes labeled using any labeling method.

11. The method for the analysis of differential expression in colorectal cancer as claimed in any one of claims 1 to 6, wherein the determination is carried out by gel electrophoresis.

12. The method for the analysis of differential expression in colorectal cancer as claimed in claim 11, wherein the determination is carried out by transfer to a membrane and hybridization with a specific probe.

13. The method for the analysis of differential expression in colorectal cancer as claimed in any one of claims 1 to 6, wherein the determination is carried out by NMR or any other diagnostic imaging technique.

14. The method for the analysis of differential expression in colorectal cancer as claimed in any one of claims 1 to 6, wherein the determination is carried out by NMR or any other diagnostic imaging technique using paramagnetic nanoparticles or any other type of detectable nanoparticles functionalized with antibodies or by any other means.

15. The method for the analysis of differential expression in colorectal cancer as claimed in any one of claims 1 to 4, wherein the sample analyzed is the protein encoded by the gene or fragments thereof.

16. The method for the analysis of differential expression in colorectal cancer as claimed in claim 15, wherein the determination is carried out by incubation with a specific antibody.

17. The method for the analysis of differential expression in colorectal cancer as claimed in claim 16, wherein the determination is carried out by Western blot.

18. The method for the analysis of differential expression in colorectal cancer as claimed in claim 16, wherein the determination is carried out by immunohistochemistry.

19. The method for the analysis of differential expression in colorectal cancer as claimed in claim 15, wherein the determination is carried out by gel electrophoresis.

20. The method for the analysis of differential expression in colorectal cancer as claimed in claim 15, wherein the determination is carried out by means of protein chips.

21. The method for the analysis of differential expression in colorectal cancer as claimed in claim 15, wherein the determination is carried out by means of ELISA or any other enzymatic method.

22. The method for the analysis of differential expression in colorectal cancer as claimed in claim 15, wherein the determination is carried out by NMR or any other diagnostic imaging technique.

23. The method for the analysis of differential expression in colorectal cancer as claimed in claim 15, wherein the determination is carried out by NMR or any other diagnostic imaging technique using paramagnetic nanoparticles or any other type of detectable nanoparticles functionalized with antibodies or by any other means.

24. The method for the analysis of differential expression in colorectal cancer as claimed in any one of the preceding claims, wherein the variation in the expression levels of the gene or genes is used to predict the progression of the colorectal cancer or of a premalignant condition thereof, or for predicting the risk of recurrence of the said disease.

25. A kit to perform the method as claimed in claims 1 to 24, comprising the requisite reagents and additives for determining the variation in the expression levels of the gene or genes.

26. A method for the analysis of compounds with therapeutic potential in colorectal cancer, comprising the determination of the capacity of said compounds to counteract the variation in the expression levels of one or more genes, as claimed in any one of claims 1 to 2, wherein the compounds are compounds tailored according to the sequence information such as antisense or RNA interference oligonucleotides or others based on the destabilization and elimination of the mRNA or the lack of its translation into protein.

27. The method for the analysis of compounds with therapeutic potential in colorectal cancer, comprising the determination of the capacity of the said compounds to counteract the variation in the expression levels of one or more genes, as claimed in claims 1 to 2, in which the compounds are paramagnetic nanoparticles or thermally-excited nanoparticles.

28. The method for the analysis of compounds with therapeutic potential in colorectal cancer, comprising the determination of the capacity of said compounds to counteract the variation in the expression levels of one or more genes, as claimed in any one of claims 1 to 2, wherein the compounds are nanoparticles functionalized with specific antibodies and toxic compounds conveyed in a simple, binary, or modular manner toward the malignant cell.

29. A pharmaceutical preparation comprising an effective amount of compounds with therapeutic potential identified by the method of any one of claims 26 to 28 and one or more pharmaceutically acceptable excipients.

30. The use of compounds with therapeutic potential identified by the method of any one of claims 26 to 28 for the preparation of a medicinal product for the treatment or prevention of colorectal cancer or any premalignant condition thereof.
